# EUROPEAN PATENT SPECIFICATION

(11) **EP 1 998 683 B1**
(45) Date of publication and mention of the grant of the patent: **07.09.2016**
(21) Application number: 07757689.0
(22) Date of filing: 01.03.2007
(51) Int. Cl.: A61B 8/14, A61B 17/22

(54) **CATHETERS**
KATHETER
CATHETERS

(30) Priority: 09.03.2006 US 780638 P
(43) Date of publication of application: 10.12.2008
(73) Proprietor: Cybersonics, Inc., Erie, PA 16510 (US)
(72) Inventor: PAL, Dharmendra, Irvine, California 92612 (US); AMATO, Michael, Peabody, Massachusetts 01960 (US); FORCUCCI, Stephen, Winchester, Massachusetts 01890 (US); CONNOR, Brian, Newfields, New Hampshire 03856 (US); HUTCHISON, Douglas, Winchester, Massachusetts 01890 (US); HAMM, Mark A., Lynnfield, Massachusetts 01940 (US)
(74) Representative: Nobbe, Matthias
(86) International application number: PCT/US2007/063039
(87) International publication number: WO 2007/103708

(56) References cited:
- WO-A1-94/12140
- US-A- 5 029 588
- US-A- 5 267 954
- US-A- 5 312 328
- US-A- 5 989 275
- US-A1- 2002 077 643
- US-A1- 2004 039 311
- US-A1- 2005 288 695

## Description

The present invention relates to catheters and related systems.

### BACKGROUND

An ultrasound medical device can be used to treat a subject (e.g., a human) having certain conditions. Typically, a portion of the ultrasound medical device is disposed within the subject, and the ultrasound medical device is activated so that the portion of the ultrasound medical device disposed within the subject vibrates at an ultrasonic frequency. The ultrasonic vibrations treat the condition by breaking up tissue in the subject.

US 5267954 discloses an ultrasound catheter for removing obstructions from tubular anatomic structures such as blood vessels, said catheter comprising an elongate flexible catheter body having an ultrasound transmission member or wire extending longitudinally therethrough. A distal head is formed on the distal end of the ultrasound transmission member or wire and is affixed to the catheter body.

US2002/077643 discloses an ultrasonic medical device operating in a transverse mode comprising a transducer capable of vibrating at an ultrasonic frequency, a probe adapted to said transducer, said probe capable of translating the vibrations from said transducer and thereby vibrating transversely along its longitude thus creating a plurality of nodes of cavitation energy along its longitude.

### SUMMARY

In general, this disclosure relates to catheters and related systems.

In more detail, the invention is directed to a system comprising:
- a catheter comprising:
   a sidewall that defines a lumen and an aperture extending around a circumference of the catheter from an outer surface of the catheter to the lumen;
   a distal portion located distal to the aperture and comprising:
      a projection that extends radially into the lumen, the projection and the side wall of the catheter opposite the projection being spaced by a distance,
      a reduced diameter portion having a diameter; and
- a waveguide disposed within the lumen, the waveguide comprising:
   a wire having a diameter; and
   a distal tip having a diameter, the distal tip being coupled with the wire and disposed in the distal portion of the catheter between the projection and the reduced diameter portion;
   wherein a portion of the waveguide is exposed via the aperture to environment exterior to the catheter;
   wherein the diameter of the distal tip is greater than the diameter of the wire; wherein the diameter of the distal tip is greater than the distance between the projection and the sidewall of the catheter opposite the projection; and
   wherein the diameter of the distal tip is greater than the diameter of the reduced diameter portion.

Thus, the inventive system includes a catheter defining a lumen and an aperture extending from an outer surface of the catheter to the lumen. The catheter includes a distal portion located distal to the aperture. A waveguide is disposed within the lumen, and a distal end region of the waveguide is disposed in the distal portion of the catheter. The catheter is configured to limit proximal movement of the waveguide relative to the catheter.

In another aspect of the invention, a system includes a catheter defining a lumen and an aperture extending from an outer surface of the catheter to the lumen. The aperture has a length of at least about five centimeters. A waveguide is disposed in the lumen, and a portion of the waveguide is exposed via the aperture to environment exterior to the catheter.

In a further aspect of the invention, a catheter defines a lumen and an aperture extending from an outer surface of the catheter to the lumen. A portion of a waveguide is exposed via the aperture to environment exterior to the catheter when the waveguide is disposed in the lumen, and the aperture has a length of at least about five centimeters.

In an additional aspect of the invention, a system includes a catheter defining a lumen and an aperture extending from an outer surface of the catheter to the lumen. A waveguide includes a distal end region that is disposed within a region of the lumen distal to the aperture. The catheter is configured to limit transverse movement of the distal end region of the waveguide to about 0.51 mm (0.020 inch) or less.

In another aspect of the invention, a system includes a catheter defining a lumen and an aperture extending from an outer surface of the catheter to the lumen. A waveguide is disposed within the lumen, and a portion of the waveguide is exposed via the aperture to environment exterior to the catheter. A sleeve is secured to a distal end region of the waveguide and to the catheter.

With the system of the invention, a method, which is not part of the invention, can be carried out which method includes disposing a portion of a system within a body vessel, where the system includes a catheter defining a lumen and an aperture extending from an outer surface of the catheter to the lumen, and the catheter is configured to limit proximal movement of a waveguide disposed within the lumen relative to the catheter. The method further includes emitting vibrational energy through the aperture by vibrating the waveguide.

Said method may include navigating a system through a body vessel, where the system includes a catheter defining a lumen and a waveguide disposed within the lumen. The method also includes emitting vibrational energy by vibrating the waveguide. The waveguide is disposed in substantially the same axial position relative to the catheter when navigating the system through the body vessel as when emitting vibrational energy.

Embodiments can include one or more of the following features.

In the inventive embodiments, a portion of the waveguide is exposed via the aperture to environment exterior to the catheter.

In the inventive embodiments, the catheter is configured to prevent a distal end of the waveguide from moving proximal to a distal end of the aperture.

In the inventive embodiments, the distal end region of the waveguide has an outer diameter that is greater than an outer diameter of a more proximal region of the waveguide.

In the inventive embodiments, the catheter includes a retention feature extending into the lumen, and the retention feature is located proximal to the distal end region of the waveguide.

In certain embodiments, the retention feature includes a projection, and the projection and an inner surface of the catheter opposite the projection are spaced by a distance that is less than the outer diameter of the distal end region of the waveguide.

In some embodiments, the retention feature comprises an annular projection extending radially inward into the lumen.

In the inventive embodiments, the catheter is configured to limit distal movement of the waveguide relative to the catheter.

In the inventive embodiments, the catheter is configured to prevent a distal end of the waveguide from moving distal to a distal end of the catheter.

In the inventive embodiments, a portion of the lumen extending within a region of the catheter located distal to the distal end region of the waveguide has a diameter that is less than the outer diameter of the distal end region of the waveguide.

In certain embodiments, the lumen is a blind lumen that terminates proximal to a distal end of the catheter.

In some embodiments, at least a portion of the lumen extending within the distal portion of the catheter has a diameter that is no more than about 0.51 mm (0.020 inch) greater than an outer diameter of the waveguide.

In certain embodiments, the aperture has a length of about five centimeters or more.

In some embodiments, the aperture is axially spaced from a distal end of the catheter by about five centimeters or less.

In the inventive embodiments, the waveguide can bow radially outward through the aperture when vibrated.

In the inventive embodiments, the catheter is configured to limit proximal movement of the waveguide relative to the catheter.

In the inventive embodiments, the catheter is configured to prevent a distal end of the waveguide from moving proximal to a distal end of the aperture.

In some embodiments, the system further includes a sleeve secured to a distal end region of the waveguide and to the catheter.

In certain embodiments, the distal end region of the waveguide is located adjacent the aperture.

In certain embodiments, the waveguide includes a portion configured to vibrate transversely during use, and the portion of the waveguide configured to vibrate transversely during use is disposed adjacent the aperture.

In some embodiments, the waveguide further includes at least one transformer section disposed in the lumen proximal to the aperture.

In certain embodiments, the system further includes a handpiece including a vibration-generating assembly, and a proximal end region of the waveguide is secured to the vibration-generating assembly.

In some embodiments, the handpiece and the waveguide are substantially axially fixed relative to the catheter.

In certain embodiments, the system further includes an adaptor securing the handpiece to the catheter, and the handpiece includes a projection disposed within an annular recess defined by the adaptor.

In some embodiments, the catheter defines a second lumen, and the second lumen has a proximal end located distal to a proximal end of the catheter.

In certain embodiments, the catheter has an outer diameter of about 0,033 mm (0.013 inch) to about 0,66 mm (0.260 inch).

In some embodiments, the aperture has a length of at least about ten centimeters. In certain embodiments, the aperture is axially spaced from a distal end of the catheter by about five centimeters or less.

In some embodiments, the region of the lumen distal to the aperture has a diameter that is at most about 0,51 mm (0.020 inch) greater than (e.g., about 0,013 mm (0.0005) inch to about 0,51 mm (0.020 inch) greater than, about 0,013 mm (0.0005 inch) to about 0,051 mm (0.002 inch) greater than) an outer diameter of the distal end region of the waveguide.

In certain embodiments, a portion of the catheter defining the region of the lumen distal to the aperture contacts the distal end region of the waveguide.

In some embodiments, the distal end region of the waveguide is encapsulated by the portion of the catheter defining the region of the lumen distal to the aperture.

In certain embodiments, the distal end region of the waveguide is located adjacent the aperture.

In the inventive embodiments, the distal end region of the waveguide is disposed in a portion of the lumen distal to the aperture.

In a method making use of the inventive system certain aspects, the method further includes rotating the catheter relative to the waveguide within the body vessel.

In a method making use of the inventive system, emitting vibrational energy through the aperture includes transversely vibrating a portion of the waveguide adjacent the aperture.

In a method making use of the inventive system, a portion of the waveguide bows outward through the aperture when the portion of the waveguide is transversely vibrated.

In a method making use of the inventive system, emitting vibrational energy through the aperture includes longitudinally vibrating a portion of the waveguide proximal to the aperture. In certain embodiments, a portion of the catheter distal to the aperture is configured to limit proximal movement of the waveguide relative to the catheter.

In the inventive embodiments, the catheter is configured to prevent a distal end of the waveguide from moving proximal to a distal end of the aperture when disposing the portion of the system within the body vessel.

In the inventive embodiments, the catheter is configured to limit distal movement of the waveguide relative to the catheter when disposing the portion of the system within the body vessel.

In the inventive embodiments, the catheter is configured to prevent a distal end of the waveguide from moving distal to a distal end of the catheter when disposing the portion of the system within the body vessel.

Embodiments can include one or more of the following advantages.

In the inventive embodiments, the distal end region of the waveguide remains disposed within the distal portion of the catheter as the system is navigated through the body vessel. This arrangement can help to prevent the waveguide (e.g., the distal end region of the waveguide) from contacting the body vessel during delivery and can help to ensure that the catheter, rather than the waveguide, absorbs compressive forces associated with navigating the system through the body vessel.

In the inventive embodiments, the distal end region of the waveguide remains disposed within the distal portion of the catheter when the waveguide is vibrated during treatment. This arrangement can help to prevent the vibrating waveguide (e.g., the distal end region of the vibrating waveguide) from contacting the body vessel during treatment.

In some embodiments, the catheter is configured to limit transverse movement of the distal end region of the waveguide to about 0,51 mm (0.020 inch) or less (e.g., about 0,013 mm (0.0005 inch) to about 0,51 mm (0.020 inch), about 0,013 mm (0.0005 inch) to about 0,051 mm (0.002 inch), about 0,0254 mm (0.001 inch). Limiting transverse movement of the distal end region of the waveguide can reduce (e.g., prevent) changes in the physical or mechanical properties of the waveguide during use.

In the inventive embodiments, the catheter is configured to limit (e.g., prevent) proximal movement of the distal end region of the waveguide with respect to the distal portion of the catheter. This arrangement can help to ensure that the waveguide (e.g., the distal end region of the waveguide) does not contact the body vessel during delivery of the system through the body vessel and during treatment of the body vessel.

In the inventive embodiments, the catheter is configured to limit (e.g., prevent) distal movement of the distal end region of the waveguide with respect to the distal portion of the catheter. This arrangement can help to ensure that the waveguide (e.g., the distal end region of the waveguide) does not contact the body vessel during delivery of the system through the body vessel and during treatment of the body vessel.

In certain embodiments, the waveguide and the catheter are longitudinally fixed relative to one another in a predetermined configuration. The proximal end regions of the waveguide and catheter can, for example, be secured to the handpiece of the system. Longitudinally fixing the waveguide and the catheter in a predetermined configuration can help to ensure that an active region of the waveguide (e.g., a region of the waveguide configured to vibrate transversely during use) is positioned adjacent the aperture of the catheter during use.

In some embodiments, the aperture is relatively long. For example the aperture can have a length of at least about five centimeters. This arrangement can help to ensure that a substantial length of an active region of the waveguide (e.g., a region of the waveguide configured to vibrate transversely during use) is exposed via the aperture to environment exterior to the catheter. In addition, the relatively long aperture can allow the waveguide to bow radially outward through the aperture when the waveguide is transversely vibrated, placing the waveguide in closer proximity to the region of the body vessel being treated. By exposing a substantial length of the active region of the waveguide via the aperture and allowing the waveguide to bow radially outward through the aperture when the waveguide is transversely vibrated, the relatively long aperture can help to ensure that treatment can be carried out at a high efficiency.

In certain embodiments, the system can be alternately moved in the proximal direction and the distal direction (e.g., alternately pushed and pulled) within a body vessel while vibrating the waveguide. This alternating movement can be performed without substantially altering the position of the waveguide relative to the catheter (e.g., without retracting the waveguide proximally into the catheter prior to moving the system in the distal direction). Thus, the system can be used to conveniently and efficiently treat a body vessel.

In some embodiments, the guide wire remains in place adjacent the active section of the waveguide during treatment. A wall of the catheter can, for example, physically separate the guide wire and the waveguide during use to prevent the vibrating waveguide from contacting the guide wire. As a result, the guide wire need not be retracted proximal to the active section of the waveguide prior to vibrating the waveguide. This can provide for a more efficient and shorter treatment.

Other aspects, features, and advantages are in the description, drawings, and claims.

### DESCRIPTION OF DRAWINGS

Figure 1 is a cross-sectional view of an ultrasonic medical system.
Figure 2 is a side view of the waveguide of the ultrasonic medical system of Figure 1.
Figure 3 is an enlarged view of region 3 in Figure 1.
Figures 4A-4D illustrate a method of using the ultrasonic medical system of Figure 1.

### DETAILED DESCRIPTION

In the invention, the systems include a catheter with a lumen that extends within the catheter and an aperture that extends from an outer surface of the catheter to the lumen. A waveguide is disposed within the lumen of the catheter, and at least a portion of the waveguide is exposed via the aperture to environment exterior to the catheter. In some embodiments, the catheter is configured to limit (e.g., prevent) proximal movement, distal movement, and/or transverse movement of a distal end region of the waveguide (e.g., a portion of the waveguide located distal to the aperture) relative to the catheter.

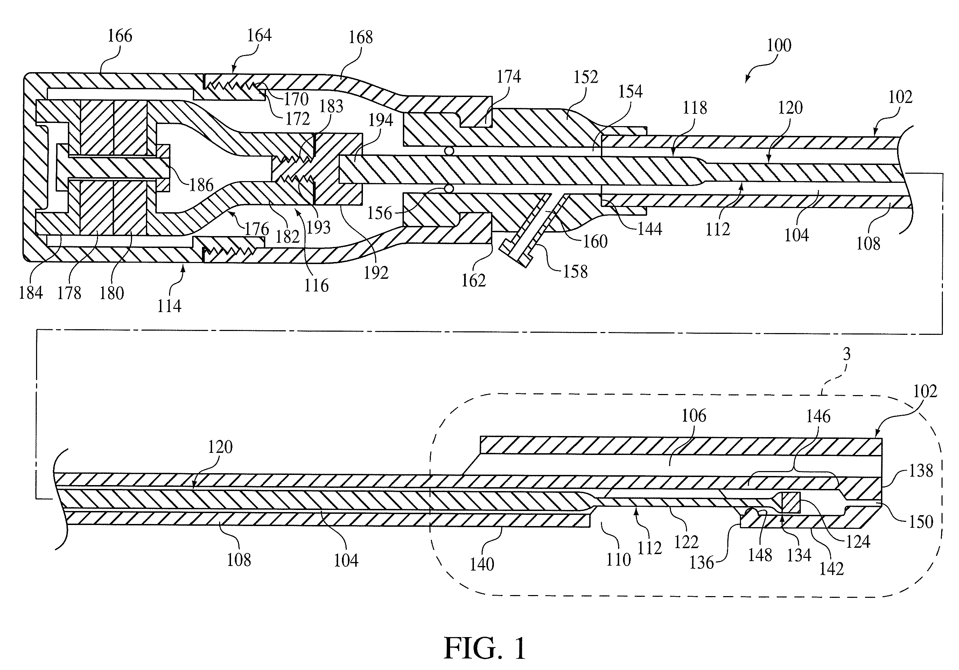

Referring to Figure 1, an ultrasonic medical system 100 includes a catheter 102 having a waveguide lumen 104 and a guide wire lumen 106. A side wall 108 of catheter 102 includes an aperture 110 that extends from the outer surface of catheter 102 inward to waveguide lumen 104. An ultrasonic probe or waveguide 112 is disposed within waveguide lumen 104 such that the portion of waveguide 112 adjacent aperture 110 is partially exposed to the environment outside of catheter 102. Proximal end regions of catheter 102 and waveguide 112 are coupled to a handpiece 114, which includes a vibration-generating assembly 116 that can be used to vibrate waveguide 112. As discussed below, during use, waveguide 112 can be vibrated, causing vibrational energy (e.g., ultrasonic vibrational energy) to be emitted via aperture 110 to environment exterior to catheter 102.

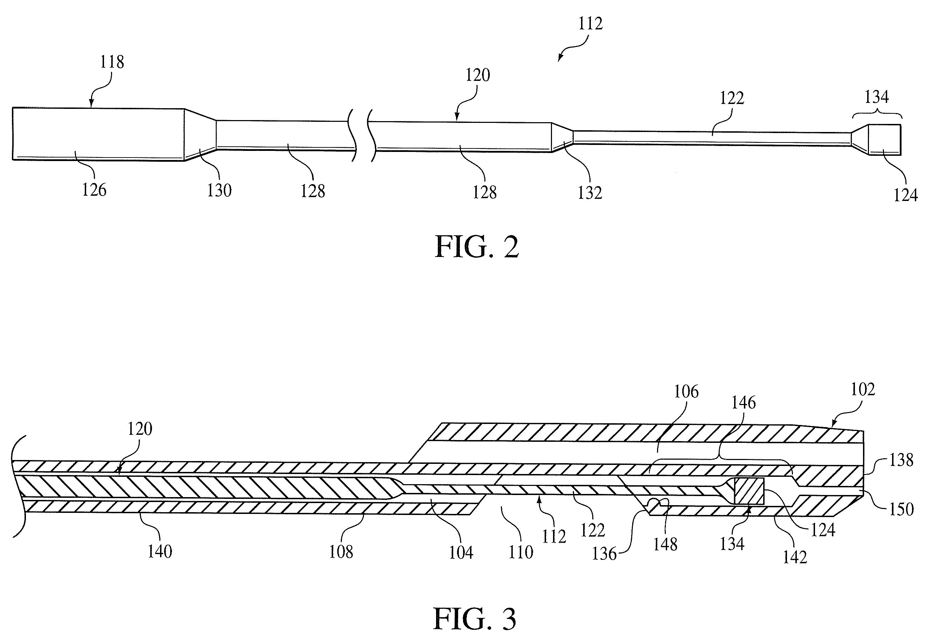

Referring to Figure 2, waveguide 112 includes a first transformer section 118, a second transformer section 120 secured to the distal end of first transformer section 118, a flexible wire 122 extending from the distal end of second transformer section 120, and a distal tip 124 secured to the distal end of flexible wire 122. First and second transformer sections 118, 120 have body portions 126, 128 and tapered portions 130, 132 that extend distally from body portions 126, 128. Tapered portions 130, 132 taper to a reduced diameter relative to their respective body portions 126, 128. Body portion 126 of first transformer section 118 has a diameter of about 0.635 mm (0.025 inch) and a length of about three centimeters. Tapered portion 130 of first transformer section 118 tapers from a diameter of about 0.635 mm (0.025 inch) at its proximal end to a diameter of about 0.43 mm (0.017 inch) at its distal end and has a length of about 12 centimeters. Body portion 128 of second transformer section 120 has a diameter of about 0.43mm (0.017 inch) and a length of about 84 centimeters. Tapered portion 132 of second transformer section 120 tapers from a diameter of about 0.43 mm (0.017 inch) at its proximal end to a diameter of about 0.25 mm (0.010 inch) at its distal end and has a length of about 12 centimeters. Flexible wire 122 has a diameter of about 0.25 mm (0.010 inch) along a majority of its length and enlarges to a diameter of about 0.40 mm (0.016 inch) near its distal end. Flexible wire 122 has a length of about ten centimeters. Distal tip 124 has a diameter of about 0.40 mm (0.016 inch).

In some embodiments, first and second transformer sections 118, 120 and flexible wire 122 are formed of 6A1-4V titanium alloy. Alternatively or additionally, first and second transformer sections 118, 120 and flexible wire 122 can include one or more other materials, such as titanium, other titanium alloys, stainless steel, and/or stainless steel alloys. First and second transformer sections 118, 120 and flexible wire 122 can be formed from a unitary rod that is ground to the desired dimensions. Alternatively, first transformer section 118, second transformer section 120, and/or flexible wire 122 can be discrete components that are secured (e.g., welded) to one another.

Distal tip 124 is formed of a highly radiopaque material, such as tantalum, platinum, iridium, and/or combinations of these materials. Distal tip 124 can be secured to the distal end of flexible wire 122 using any of various techniques, such as welding, thermally bonding, etc. During use, distal tip 124 can be used to help position waveguide 112 as desired within a blood vessel by, for example, using an imaging technique, such as fluoroscopy.

Due to the configuration and materials of waveguide 112, a longitudinal vibration applied to the proximal end of waveguide 112 (e.g., to the proximal end of first transformer section 118 of waveguide 112) can be amplified by first and second transformer sections 118, 120, and the amplified longitudinal vibration can be transferred to flexible wire 122, causing flexible wire 122 to buckle. As a result, a standing transverse wave can be created along flexible wire 122. The standing transverse wave can create multiple nodes and anti-nodes of transverse vibration along flexible wire 122.

A distal end region 134 of waveguide 112 is made up of distal tip 124 and the enlarged distal end of flexible wire 122. Distal end region 134 has a diameter that is greater (e.g., about 0.15 mm (0.006 inch) greater) than the portion of waveguide 112 immediately proximal to distal end region 134.

Referring again to Figure 1, aperture 110 of catheter 102 can be sized to permit a desired amount of vibrational energy resulting from the vibration of waveguide 112 (e.g., from the transverse vibration of flexible wire 122 of waveguide 112) to pass through aperture 110. Aperture 110 can, for example, have a length of at least about one centimeter (e.g., at least about five centimeters, at least about 10 centimeters, at least about 15 centimeters, at least about 20 centimeters, at least about 25 centimeters). In certain embodiments, aperture 110 has a length of about one centimeter to about 30 centimeters (e.g., about five centimeters to about 30 centimeters, about seven centimeters to about 15 centimeters, about 12 centimeters). In some embodiments, aperture 110 extends about 90 degrees or more (e.g., about 90 degrees to about 270 degrees) around the circumference of waveguide lumen 104.

A distal end 136 of aperture 110 is located in relatively close proximity to a distal end 138 of catheter 102 and to distal end region 134 of waveguide 112. In some embodiments, for example, distal end 136 of aperture 110 is located about five centimeters or less (e.g., about one centimeter to about five centimeters, about 2.5 centimeters) from distal end 138 of catheter 102. Locating distal end 136 of aperture 110 in close proximity to distal end 138 of catheter 102 and in close proximity to distal end region 134 of waveguide 112 can help to ensure that a substantial portion of flexible wire 122 of waveguide 112 is exposed to the environment exterior to catheter 102 via aperture 110. This can help to increase the amount of vibrational energy resulting from transverse vibration of flexible wire 122 that is emitted through aperture 110 during use.

Still referring to Figure 1, catheter 102 includes a proximal portion 140 located proximal to aperture 110 and a distal portion 142 located distal to aperture 110. Waveguide lumen 104 extends through both proximal and distal portions 140, 142 of catheter 102, from a proximal end 144 of catheter 102 to distal end 138 of catheter 102.

Referring to Figure 3, distal end region 134 of waveguide 112 is disposed within a region 146 of waveguide lumen 104, which extends within distal portion 142 of catheter 102. Region 146 of waveguide lumen 104 has a diameter of at most 0.51 mm (0.020 inch) greater than (e.g., about 0.013 mm (0.0005 inch) to about 0.51 mm (0.020 inch) greater than, about 0.013 mm (0.0005 inch) to about 0.051 mm (0.002 inch) greater than, about 0.025 mm (0.001 inch) greater than) the diameter of distal end region 134 of waveguide 112. Region 146 of waveguide lumen 104 can have a length of about one centimeter to about ten centimeters (e.g., about two centimeters).

The configuration of region 146 of waveguide lumen 104 can reduce (e.g., minimize) transverse movement of distal end region 134 of waveguide 112, while permitting distal end region 134 of waveguide 112 to slide axially within region 146 of waveguide lumen 104. The configuration of region 146 of waveguide lumen 104 can, for example, reduce transverse movement of distal end region 134 of waveguide 112 to about 0.51 mm (0.020 inch) or less (e.g., about 0.013 mm (0.0005 inch) to about 0.51 mm (0.020 inch), about 0.013 mm (0.0005 inch) to about 0.051 mm (0.002 inch), about 0.025 mm (0.001 inch)). Restricting transverse movement of distal end region 134 of waveguide 112 can help to maintain stress levels in waveguide 112 within a desirable or acceptable range. The stress levels can, for example, be maintained within a range in which the physical properties of waveguide 112 remain substantially unchanged during use. At the same time, allowing distal end region 134 of waveguide 112 to slide axially along the length of region 146 of waveguide lumen 104 can facilitate navigation of system 100 through a blood vessel by, for example, decreasing resistance experienced by system 100 when catheter 102 and waveguide 112 are navigated around bends within the blood vessel.

As shown in Figure 3, distal portion 142 of catheter 102 includes a projection 148 that extends radially from side wall 108 into waveguide lumen 104 at the proximal end of region 146 of waveguide lumen 104. Projection 148 is located proximal to distal end region 134 of waveguide 112. Projection 148 and the side wall of catheter 102 opposite projection 148 are spaced apart by a distance that is less than the diameter of distal end region 134 of waveguide 112. Projection 148 can, for example, extend about 0.013 mm (0.0005 inch) to about 0.076 mm (0.003 inch) (e.g., about 0.025 mm (0.001 inch)) radially into waveguide lumen 104. Because projection 148 and the side wall of catheter 102 opposite projection 148 are spaced apart by a distance that is less than the diameter of distal end region 134 of waveguide 112, when waveguide 112 is moved proximally relative to catheter 102 to the proximal end of region 146 of waveguide lumen 104, distal end region 134 of waveguide 112 contacts projection 148, preventing further proximal movement of waveguide 112 relative to catheter 102. As a result, distal end region 134 of waveguide 112 can be prevented from extending into the portion of waveguide lumen 104 adjacent aperture 110 during use.

Projection 148 can be integrally formed with the side wall 108 of catheter 102. Projection 148 can, for example, be formed by pressing a hot knife radially against the outer surface of catheter 102. Such a technique forms a depression in the outer surface of catheter 102, causing projection 148 to extend radially into waveguide lumen 104. Alternatively or additionally, any of various other suitable techniques can be used to form projection 148. For example, a mandrel having a portion with an outer diameter that is smaller than the outer diameter of the distal tip 124 of waveguide 112 can be disposed within a lumen of a catheter tube and a heat shrink tube can be disposed around an outer surface of the catheter tube, and then the assembly can be heated such that the portion of the lumen surrounding small diameter portion of the madrel is reduced to a diameter that is less than the diameter of distal tip 124.

Still referring to Figure 3, waveguide lumen 104 includes a reduced diameter portion 150 located distal to waveguide 112. Reduced diameter portion 150 extends distally from the distal end of region 146 of waveguide lumen 104. Reduced diameter portion 150 has a diameter that is less than the diameter of distal end region 134 of waveguide 112. In some embodiments, reduced diameter portion 150 of waveguide lumen 104 has a diameter of about 0.025 mm (0.010 inch) to about 0.063 mm (0.025 inch) (e.g., about 0.381 mm (0.015 inch)). Because the diameter of reduced diameter portion 150 of waveguide lumen 104 is less than the diameter of distal end region 134 of waveguide 112, waveguide 112 is prevented from extending into reduced diameter portion 150 of waveguide lumen 104 during use. For example, when waveguide 112 is slid axially to the distal end of region 146 of waveguide lumen 104, distal tip 124 of waveguide 112 contacts the portion of catheter 102 that forms reduced diameter portion 150 of waveguide lumen 104, preventing further distal movement of waveguide 112 relative to catheter 102. As a result, waveguide 112 can be prevented from extending distally beyond distal end 138 of catheter 102 during use.

Because distal end region 134 of waveguide 112 is prevented from extending into the portion of waveguide lumen 104 adjacent aperture 110 and is prevented from extending distally beyond distal end 138 of catheter 102, the distal end of waveguide 112 can be prevented from contacting a blood vessel wall during delivery of system 100 through a blood vessel and during treatment of the blood vessel using system 100.

Referring again to Figure 1, guide wire lumen 106 of catheter 102, which extends along side a distal region of waveguide lumen 135, is substantially shorter than waveguide lumen 135 of catheter 102. In some embodiments, guide wire lumen 106 has a length of about one centimeter to about 50 centimeters. Guide wire lumen 106 can, for example, have a length of about 25 centimeters. Guide wire lumen 106 is configured to allow a guide wire to be threaded through guide wire lumen 106. In certain embodiments, for example, guide wire lumen 106 has a diameter of about 0.025 mm (0.010 inch) to about 0.762 mm (0.030 inch) (e.g., about 0.56 mm (0.022 inch)).

Catheter 102 can be any of various different sizes, depending on its intended use. In general, catheter 102 can have an outer diameter of about 0.33 mm (0.013 inch) to about 6.6 mm (0.260 inch) and/or a length of about 25 centimeters to about 150 centimeters. In some embodiments, catheter 102 is sized for use in a femoral artery. In such embodiments, catheter 102 can have an outer diameter of about 1.3 mm (0.052 inch) to about 1.98 mm (0.078 inch) and a length of about 80 centimeters to about 100 centimeters. In certain embodiments, catheter 102 is sized for use in neuro blood vessels, in which case catheter 102 can have an outer diameter of about 0.66 mm (0.026 inch) to about 0.99 mm (0.039 inch) and a length of about 25 centimeters to about 60 centimeters.

In some embodiments, catheter 102 is formed of multiple different materials along its length. For example, catheter 102 can be formed of multiple different materials along its length so that the durometer of catheter 102 decreases from its proximal end to its distal end such that catheter 102 is more flexible near its distal end than near its proximal end. In such embodiments, catheter 102 can be constructed of multiple longitudinal segments of differing durometer that are attached (e.g., bonded) to one another to form catheter 102. In some embodiments, for example, catheter 102 includes polyether block amides (e.g., PEB AX^{®}) of differing durometers. Any of various manufacturing techniques, such as extrusion and/or injection molding, can be used to manufacture the longitudinal segments of catheter 102.

As an alternative to being formed of multiple segments, catheter 102 can be formed as a unitary member, for example, using coextrusion techniques. Moreover, while catheter 102 has been described has including multiple different materials of differing durometer, catheter 102 can alternatively be formed of a single, relatively flexible material, such as a polyether block amide of a desired durometer.

Still referring to Figure 1, an adaptor 152 is secured to catheter 102 near proximal end 144 of catheter 102. In some embodiments, adaptor 152 is ultrasonically welded to catheter 102. Adaptor 152 can alternatively or additionally be secured to catheter 102 using one or more other techniques, such as thermal bonding, adhesive bonding, and/or mechanical fastening. Adaptor 152 includes a central lumen 154 that is aligned with waveguide lumen 104 of catheter 102. An O-ring 156 is disposed within central lumen 154 to prevent leakage of blood or other body fluids into handpiece 114 during use. Adaptor 152 also includes a luer lock fitting 158 that defines a port 160 that is in fluid communication with central lumen 154. An annular recess 162 is formed in the outer surface of adaptor.

Handpiece 114 includes a housing assembly 164 that includes a main body portion 166 and a nosecone portion 168. Nosecone portion 168 includes threads 170 that mate with threads 172 on a distal end region of main body portion 166 to secure nosecone potion 168 to main body portion 166. Nosecone portion 168 is tapered from its proximal end to its distal end. The distal end region of nosecone portion includes an annular, inwardly extending projection 174 that is disposed within annular recess 162 of adaptor 152. Nosecone portion 168 can be formed of a resilient material such that, when nosecone portion 168 is slid onto adaptor 152, the distal end region of nosecone portion 168 deflects outward and, upon reaching annular recess 162 of adaptor 152, annular projection 174 of nosecone portion 168 snaps into annular recess 162. Annular recess 162 of adaptor 152 and annular projection 174 of nosecone portion 168 cooperate to longitudinally fix handpiece 114 to adaptor 152 while allowing adaptor 152 to rotate relative to handpiece 114. Because adaptor 152 is fixed to catheter 102, catheter 102 is similarly longitudinally fixed relative to handpiece 114 and rotatable relative to handpiece 114.

Vibration-generation assembly 116 includes an ultrasonic horn 176 having a front portion 182 and a back mass. Two piezeoceramic rings 178, 180 are disposed between front portion 182 and back mass 184 of horn 176. Piezeoceramic rings 178, 180 are held tightly together between front portion 182 and back mass 184 of horn 176 by a bolt 186 extending through central apertures of piezeoceramic rings 178, 180. Front portion 182 of horn 176 includes a threaded region 183 that is used to secure front portion 182 of horn 176 to waveguide 112. Back mass 184 of horn 176 is secured (e.g., bonded) to the proximal end of main body portion 166 of housing assembly 164. As a result, horn 176 is axially fixed relative to housing assembly 164 of handpiece 114.

During use, piezeoceramic rings 178, 180 are electrically connected to an electrical power supply (not shown). Piezeoceramic rings 178, 180 are configured so that, when electrical energy is received from the power supply, piezeoceramic rings 178, 180 vibrate (e.g., ultrasonically vibrate) in a longitudinal direction. The vibrational energy emitted by piezeoceramic rings 178, 180 causes horn 176 to similarly vibrate in a longitudinal direction.

Still referring to Figure 1, a threaded coupler 192 is attached (e.g., welded) to a proximal end region 194 of waveguide 112. Threaded coupler 192 includes threads 193 that are matingly secured to threads on threaded region 183 of front portion 182 of horn 176. Thus, horn 176, when vibrated in a longitudinal direction, causes waveguide 112 to vibrate in a longitudinal direction. Flexible wire 122 of waveguide 112, as discussed above, is configured to vibrate transversely when longitudinal vibration is transferred to waveguide 112 by horn 176. In particular, when longitudinal vibrational energy is transferred to first transformer section 118 by horn 176, first transformer section 118 amplifies the longitudinal vibration, and the amplified longitudinal vibration is transferred to second transformer section 120. Second transformer section 120 further amplifies the longitudinal vibration, which is then transferred to flexible wire 122. This longitudinal vibrational energy causes flexible wire 122 to buckle, creating a standing transverse vibrational wave that extends along flexible wire 122.

Waveguide 112 is axially fixed to handpiece 114 by vibration-generating assembly 116. As noted above, catheter 102 is also axially fixed to handpiece 114 by adaptor 152. As a result, waveguide 112 and catheter 102 can be axially secured relative to one another in a predetermined configuration. For example, catheter 102 and waveguide 112 can be configured so that the active region of waveguide 112 (e.g., flexible wire 122, which vibrates transversely during use) is located adjacent aperture 110 and transformer sections 118, 120 are located proximal to aperture 110. The configuration of catheter 102 allows waveguide 112 and catheter 102 to remain substantially axially fixed relative to one another throughout use. Because aperture 110 permits vibrational energy transmitted by waveguide 112 to pass through side wall 108 of catheter 102, waveguide 112 can be positioned in substantially the same position relative to catheter 102 when delivered through a patient's blood vessel and when vibrated to treat the patient's blood vessel. For example, waveguide 112 need not be extended distally beyond the distal end of catheter 102 while treating the blood vessel and waveguide 112 need not be retracted proximally relative to catheter 102 prior to being delivered through the blood vessel.

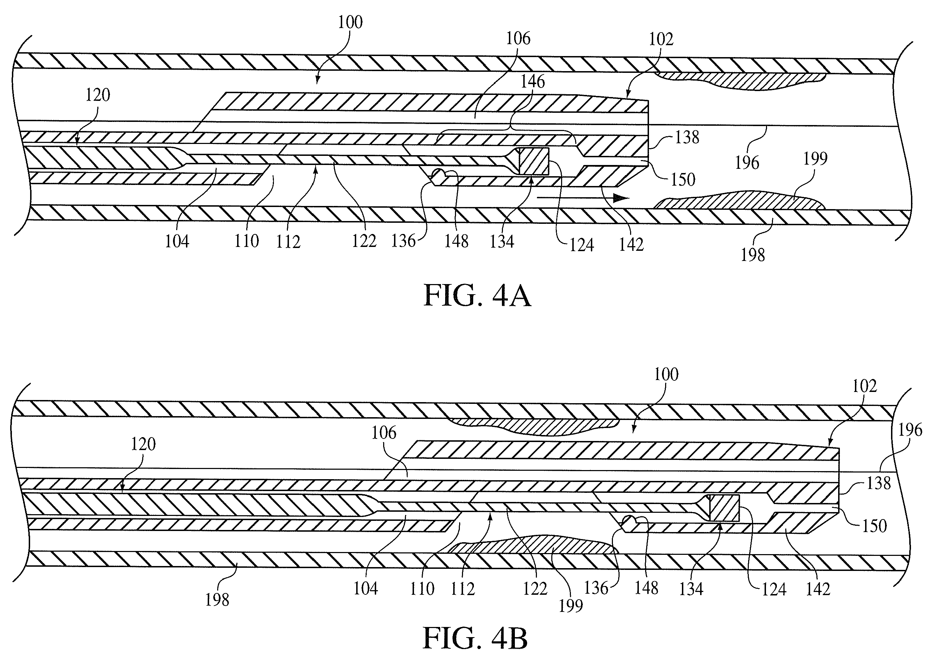

Figures 4A-4D illustrate a method of using ultrasonic medical system 100. Referring to Figure 4A, after disposing a guide wire (e.g., a conventional 0.45 mm (0.018 inch) diameter guide wire) 196 within a blood vessel 198, catheter 102 is threaded over guide wire 196 and through blood vessel 198. Catheter 102 is guided toward an occluded region 199 of blood vessel 198. As catheter 102 is navigated through blood vessel 198, guide wire 196 is disposed within guide wire lumen 106 of catheter 102, which helps to guide catheter 102 through the vessel. Distal end region 134 of waveguide 112 is disposed within region 146 of waveguide lumen 104, which extends within distal portion 142 of catheter 102, as catheter 102 is navigated through blood vessel 198. When catheter 102 is navigated through tortuous regions of blood vessel 198, catheter 102 tends to bend, which can cause waveguide 112 to move proximally relative to catheter 102. Projection 148, however, prevents distal end region 134 of waveguide 112 from moving proximal to distal end 136 of aperture 110. Similarly, reduced diameter portion 150 of waveguide lumen 104 prevents waveguide 112 from moving distal to distal end 138 of catheter 102. Limiting proximal and distal movement of waveguide 112 helps to ensure that distal end region 134 of waveguide 112 remains disposed within distal portion 142 of catheter 102 during delivery. As a result, distal end region 134 of the waveguide 112 can be prevented from contacting the wall of blood vessel 198 during delivery. This can help to ensure that catheter 102, rather than waveguide 112, absorbs compressive forces associated with navigating catheter 102 and waveguide 112 through blood vessel 198.

Referring to Figure 4B, catheter 102 and waveguide 112 are navigated through blood vessel 198 until aperture 110 of catheter 102 is positioned adjacent occluded region 199 of blood vessel 198. Any of various imaging techniques, such as fluoroscopy, can be used to ensure that aperture 110 of catheter 102 and the portion of waveguide 112 adjacent aperture 110 are disposed within occluded region 199 of blood vessel 198. One or more of these imaging techniques can, for example, be used to make sure that radiopaque distal tip 124 of waveguide 112 is positioned slightly distal to occluded region 199, which can indicate that aperture 110 is positioned adjacent or within occluded region 199.

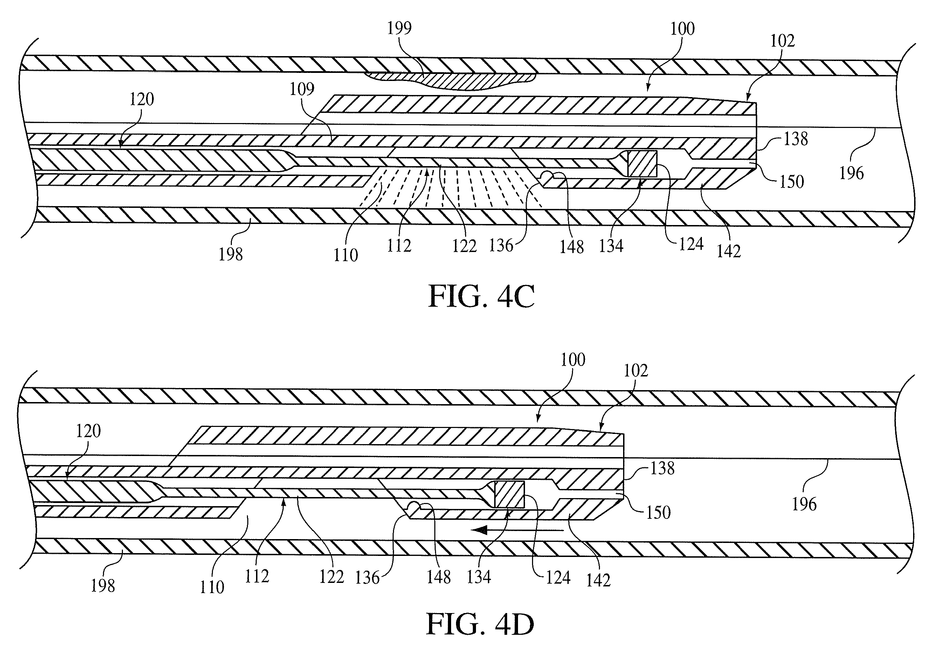

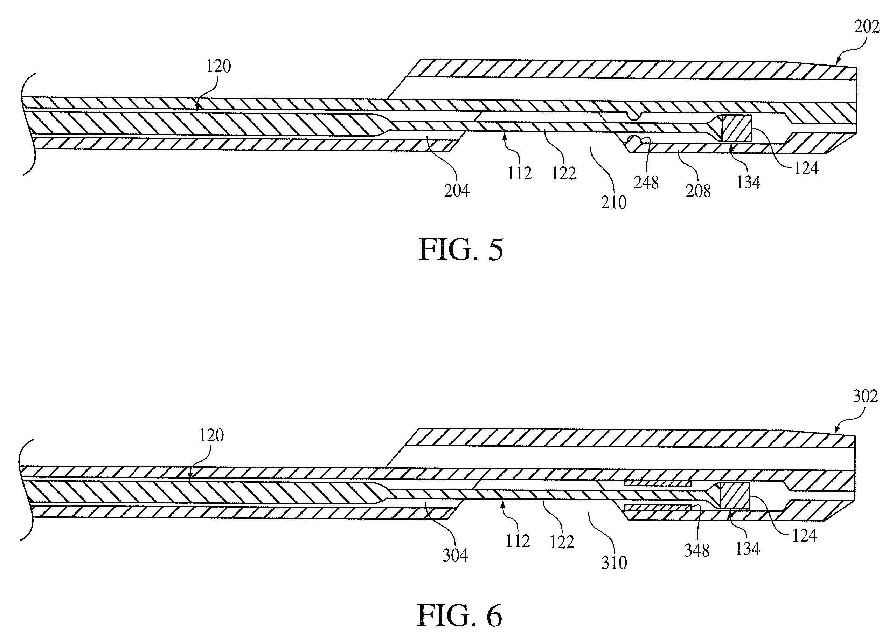

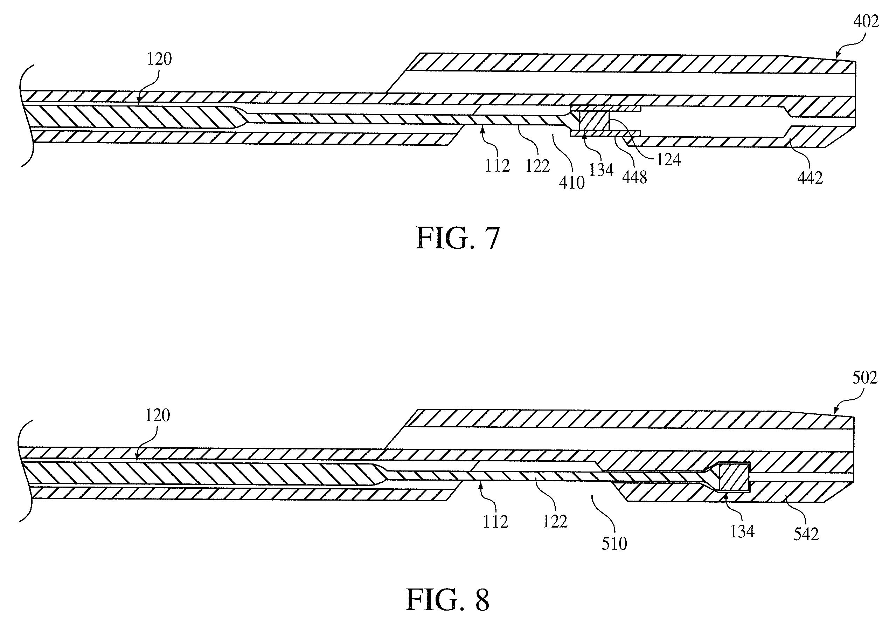

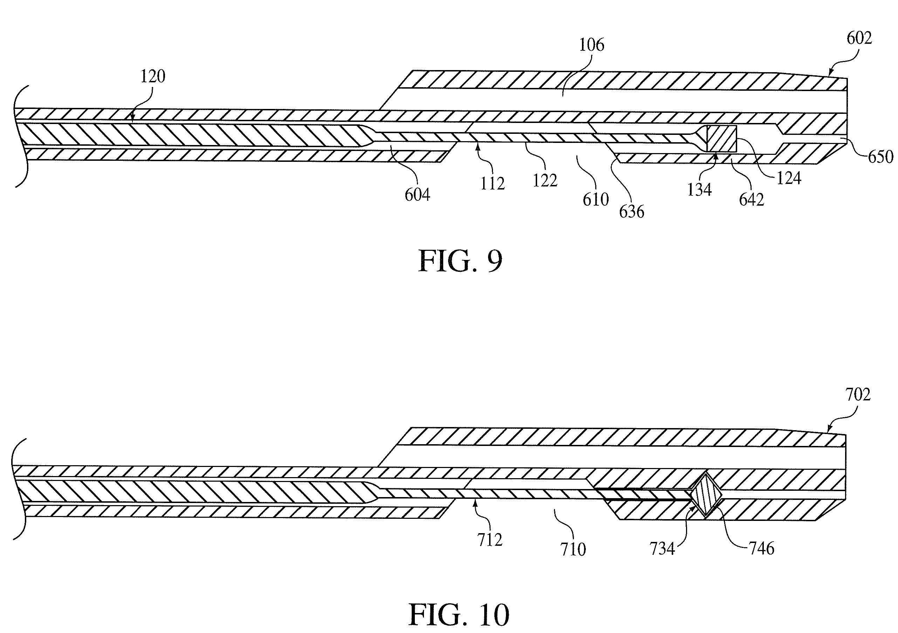

Referring to Figure 4C, after positioning catheter 102 and waveguide 112 as desired within occluded region 199 of blood vessel 198, waveguide 112 is activated (e.g., by supplying electrical energy to vibration-generation assembly 116), causing waveguide 112 to vibrate (e.g., vibrate ultrasonically). Waveguide 112 can, for example, be vibrated at a frequency of about 20 kHz to about 100 kHz. Because the active region of waveguide 112 (e.g., flexible wire 122 of waveguide 112) is positioned adjacent aperture 110 during both delivery and treatment, the physician does not typically need to reposition waveguide 112 relative to catheter 102 prior to activating waveguide 112. Vibrational energy emitted by the portion of waveguide 112 adjacent aperture 110 (e.g., by flexible wire 122 of waveguide 112) passes through aperture 110 and contacts occluded region 199 of blood vessel 198. This vibrational energy acts on occluded region 199, causing occluded region 199 to break apart into small particles.

Because distal end region 134 of waveguide 112 is enclosed within distal portion 142 of catheter 102, distal end region 134 of waveguide 112 is prevented from contacting the wall of blood vessel 198 during treatment. Similarly, during treatment, waveguide 112 is prevented from contacting guide wire 196 by a wall 109 of catheter 102 that physically separates waveguide lumen 104 from guide wire lumen. Thus, the physician does not typically need to retract or remove guide wire 196 prior to activating waveguide 112.

Due to the size of aperture 110 relative to the size of flexible wire 122 of waveguide 112, as waveguide 112 is vibrated, a portion of flexible wire 122 can bow radially outward through aperture 110. The proximity of flexible wire 122 relative to the wall of blood vessel when flexible wire 122 bows outward through aperture 110 can result in occluded region 199 being treated with vibrational energy of increased intensity, as compared to treatments in which a waveguide remains entirely within a catheter during treatment. Thus, this arrangement can increase speed and efficiency of the treatment performed to remove occluded region 199.

While vibrating waveguide 112, catheter 102 is rotated to expose the portion of waveguide 112 adjacent aperture 110 (e.g., flexible wire 122 of waveguide 112) to various different regions (e.g., circumferentially spaced regions) of blood vessel 198, allowing the various different regions within blood vessel 198 to be treated. In some embodiments, for example, catheter 102 is rotated 360 degrees. This can help to ensure that occluded region 199 of blood vessel 198 is removed from substantially the entire inner circumference of blood vessel 198. The physician can also move system 100 back and forth (forward and backward) through occluded region 199 during use.

In some embodiments, during use, a cooling and/or lubricating fluid is passed through waveguide lumen 104. The fluid can, for example, be injected into waveguide lumen 104 via luer lock fitting 158 of adaptor 152. The fluid can help to maintain the temperature of waveguide 112 within a desired or acceptable temperature range during treatment. Alternatively or additionally, a radiopaque contrast fluid can be passed through waveguide lumen 104 during use.

Referring to Figure 4D, after treating (e.g., removing) occluded region 199, catheter 102, waveguide 112, and guide wire 196 are removed from blood vessel 198.

Blood vessel 198 can be any of various different types of blood vessels. For example, blood vessel 198 can be a femoral blood vessel (e.g., a femoral artery) or a neuro blood vessel.

As a further example, while catheters of certain embodiments discussed herein are described as including a guide wire lumen extending along side only a distal portion of a waveguide lumen, the guide wire lumen can alternatively or additionally extend along side other regions of the waveguide lumen. For example, the guide wire lumen can extend along side proximal and/or central regions of the waveguide lumen. In some embodiments, the guide wire lumen extends along side substantially the entire length of the waveguide lumen.

As another example, while certain embodiments have been described in which the catheter includes a waveguide lumen and a guide wire lumen, the catheter can include fewer or greater lumens. In some embodiments, for example, the catheter includes only a waveguide lumen. In certain embodiments, in addition to the waveguide lumen and the guide wire lumen, the catheter includes an aspiration lumen and/or a flushing lumen.

As an additional example, while waveguide 112 has been described as including an active section that vibrates in the transverse direction, waveguide 112 can alternatively or additionally be configured so that the active region vibrates in a longitudinal and/or torsional direction.

As another example, while waveguide 112 has been described as having certain dimensions, waveguide can have any of various different dimensions that allow waveguide to vibrate in a desired manner. Flexible wire can, for example, have a diameter of about 0.051 mm (0.002 inch) to about 1.02 mm (0.040 inch) (e.g., about 0.010 mm (0.004 inch) to about 0.43 mm (0.017 inch)) and a length of about ten centimeters to about 200 centimeters (e.g., about 60 centimeters to about 110 centimeters). Distal end region 134 of waveguide 112 can have a diameter of about 0.051 mm (0.002 inch) to about 0.51 mm (0.020 inch) (e.g., about 0.10 mm (0.004 inch) to about 0.25 mm (0.010 inch)) and a length of about 0.5 centimeter to about 20 centimeters (e.g., about one centimeter to about ten centimeters). Any of the various other parts of waveguide 112 can similarly have different dimensions depending, for example, on the intended use of waveguide 112.

While the catheter of certain embodiments discussed above has been described as being rotatable relative to the handpiece, in some embodiments, the catheter is rotationally fixed relative to the handpiece. In certain embodiments, for example, the adaptor that secures the handpiece to the catheter is rotationally fixed relative to both the catheter and the handpiece. The adaptor can, for example, be welded (e.g., ultrasonically welded) to both the catheter and the handpiece.

While adaptor 152 and catheter 102 have been described as being axially fixed to nosecone portion 168 of housing assembly 164 of handpiece 114 using a snap fitting technique, other coupling techniques can alternatively or additionally be used. In some embodiments, for example, nosecone portion 168 is welded (e.g., ultrasonically welded) to adaptor 152. Other examples of coupling techniques include telescopic connections, threaded connections, etc.

While vibration-generating assembly 116 has been described as including piezoceramic rings 178, 180, other types of transducers can alternatively or additionally be used. For example, transducers including one or more other types of materials, such as magnetostrictive materials, can be used. As another example, transducers of other shapes, such as cylindrical transducers and disk-shaped transducers can alternatively or additionally be used.

While system 100 has been described as being used to remove an occluded region of a vessel (e.g., a region occluded with plaque), system 100 can alternatively or additionally be used to perform other types of treatment. For example, system 100 can alternatively or additionally be used to treat (e.g., remove) other types of biological material, such as tissue, cysts, tumors, etc. While system 100 has been described as being used to perform treatments in various different types of blood vessels, system 100 can alternatively or additionally be used to perform treatments in other types of body vessels or body parts, such as biliary vessels, urethras, uterus, prostates, esophagus, intestines, lymph system, pleural space, sinus. System 100 can similarly be use to perform treatments in other natural orificies, such as ear canals, eye sockets, and the like.

Other embodiments are in the claims.

## Claims

1. A system, comprising:
- a catheter (102) comprising:
a sidewall (108) that defines a lumen (104) and an aperture (110) extending around a circumference of the catheter (102) from an outer surface of the catheter (102) to the lumen (104);
a distal portion (142) located distal to the aperture and comprising:
a projection (148) that extends radially into the lumen (104), the projection (148) and the side wall (108) of the catheter (102) opposite the projection (148) being spaced by a distance,
a reduced diameter portion (150) having a diameter extending distally from a distal end of portion (146) of the lumen (104); and
- a waveguide (112) disposed within the lumen (104), the waveguide comprising:
a wire (122) having a diameter; and
a distal tip (124) having a diameter, the distal tip (124) being coupled with the wire (122) and disposed in the distal portion of the catheter (102) between the projection (148) and the reduced diameter portion (150);
- wherein a portion of the waveguide (112) is exposed via the aperture (110) to environment exterior to the catheter (102);
- wherein the diameter of the distal tip (124) is greater than the diameter of the wire (122);
- wherein the diameter of the distal tip (124) is greater than the distance between the projection (148) and the sidewall (108) of the catheter (102) opposite the projection (148) ; and
- wherein the diameter of the distal tip (124) is greater than the diameter of the reduced diameter portion (150).

2. The system of claim 1, wherein at least a portion of the lumen (104) extending within the distal portion of the catheter (102) has a diameter that is not more than about 0.51 millimeters (0,020 inch) greater than the outer diameter of the distal tip.

3. The system of claim 1, wherein the aperture (110) has a length of about five centimeters or more.

4. The system of claim 1, wherein the aperture (110) is axially spaced from a distal end of the catheter (102) by about five centimeters or less.

5. The system of claim 1, wherein the catheter (102) is axially fixed in a predetermined configuration relative to the waveguide.

## Patentansprüche

1. Ein System umfassend:
- einen Katheter (102) umfassend:
eine Seitenwand (108), die definiert ein Lumen (104) und eine Öffnung (110), die sich entlang eines Umfangs des Katheters (102) von einer äußeren Oberfläche des Katheters (102) bis zum Lumen (104) erstreckt;
einen distalen Abschnitt (142), der distal zu der Öffnung angeordnet ist und umfasst:
einen Vorsprung (148), der sich radial in das Lumen (104) erstreckt, wobei der Vorsprung (148) und die dem Vorsprung gegenüberliegende Seitenwand (108) des Katheters (102) in einem Abstand angeordnet ist,
einen Abschnitt mit verringertem Durchmesser (150), dessen Durchmesser sich distal von einem distalen Ende des Abschnitts (146) des Lumens (104) erstreckt, und
- einen Wellenleiter (112), der innerhalb des Lumens (104) angeordnet ist, wobei der Wellenleiter umfasst:
einen Draht (122) mit einem Durchmesser; und
eine distalen Spitze (124) mit einem Durchmesser, wobei die distale Spitze (124) mit dem Draht (122) gekoppelt ist und in dem distalen Abschnitt des Katethers (102) zwischen dem Vorsprung (148) und dem Abschnitt mit verringertem Durchmesser (150) angeordnet ist,
- wobei ein Abschnitt des Drahts (122) über die Öffnung (110) der Umgebung außerhalb des Katheters (102) ausgesetzt ist;
- wobei der Durchmesser der distalen Spitze (124) größer ist als der Durchmesser des Drahtes (122);
- wobei der Durchmesser der distalen Spitze (124) größer ist als der Abstand zwischen dem Vorsprung (148) und der dem Vorsprung (148) gegenüberliegenden Seitenwand (108) des Katheters (102); und
- wobei der Durchmesser der distalen Spitze (124) größer ist als der Durchmesser des Abschnitts mit verringertem Durchmesser (150).

2. Das System nach Anspruch 1, wobei mindestens ein Abschnitt des Lumens (104), das sich im distalen Teil des Katheters erstreckt, einen Durchmesser hat, der nicht mehr als 0,51 Millimeter (0,020 Inch) größer als der äußere Durchmesser der distalen Spitze ist.

3. Das System nach Anspruch 1, wobei die Öffnung (110) eine Länge von etwa fünf Zentimeter oder mehr aufweist.

4. Das System nach Anspruch 1, wobei die Öffnung (110) axial mit einem Abstand von etwa fünf Zentimetern oder weniger vom distalen Ende des Katheters (12) angeordnet ist.

5. Das System nach Anspruch 1, wobei der Katheter (102) axial in einer bezogen auf den Wellenleiter vorgegeben Konfiguration fixiert ist.

## Revendications

1. Système, comprenant :
- un cathéter (102) comprenant :
une paroi latérale (108) qui définit une lumière (104) et une ouverture (110) s'étendant autour d'une circonférence du cathéter (102) depuis la surface extérieure du cathéter (102) jusqu'à la lumière (104) ;
une partie distale (142) localisée de manière distale par rapport à l'ouverture et comprenant :
une protubérance (148) qui s'étend radialement dans la lumière (104), la protubérance (148) et la paroi latérale (108) du cathéter (102) en face de la protubérance (148) étant séparées d'une certaine distance,
une partie à diamètre réduit (150) ayant un certain diamètre s'étendant de manière distale depuis l'extrémité distale de la partie (146) de la lumière (104), et
- un guide d'ondes (112) disposé à l'intérieur de la lumière (104), le guide d'ondes comprenant :
un fil (122) ayant un certain diamètre ; et
un embout distal (124) ayant un certain diamètre, l'embout distal (124) étant couplé au fil (122) et étant disposé dans la partie distale du cathéter (102) entre la protubérance (148) et la partie à diamètre réduit (150) ;
- où une partie du guide d'ondes (112) est exposée via l'ouverture (110) à l'environnement extérieur au cathéter (102) ;
- où le diamètre de l'embout distal (124) est supérieur au diamètre du fil (122) ;
- où le diamètre de l'embout distal (124) est supérieur à la distance entre la protubérance (148) et la paroi latérale (108) du cathéter (102) en face de la protubérance (148) ; et
- où le diamètre de l'embout distal (124) est supérieur au diamètre de la partie à diamètre réduit (150).

2. Système selon la revendication 1, dans lequel au moins une partie de la lumière (104) s'étendant à l'intérieur de la partie distale du cathéter (102) a un diamètre qui n'est pas supérieur de plus de 0,51 millimètres environ (0,020 pouce) au diamètre de l'embout distal.

3. Système selon la revendication 1, dans lequel l'ouverture (110) a une longueur d'environ cinq centimètres ou plus.

4. Système selon la revendication 1, dans lequel l'ouverture (110) est axialement séparée de l'extrémité distale du cathéter (102) d'environ cinq centimètres ou moins.

5. Système selon la revendication 1, dans lequel le cathéter (102) est axialement fixé dans une configuration prédéterminée par rapport au guide d'ondes.
